# EUROPÄISCHE PATENTANMELDUNG

(11) **EP 1 201 210 A1**
(43) Veröffentlichungstag der Anmeldung: **02.05.2002**
(21) Anmeldenummer: 01810979.3
(22) Anmeldetag: 09.10.2001
(51) Int. Cl.: A61F 9/007

(54) **Einrichtung zur Durchführung ophthalmologischer Eingriffe**

(30) Priorität: 23.10.2000 US 694214
(71) Anmelder: Alcon Grieshaber AG, 8203 Schaffhausen (CH)
(72) Erfinder: Attinger, Jürg, 8260 Stein am Rhein (CH); Maag, Werner, 8750 Glarus (CH); Egli, Lorenz, 8212 Neuhausen (CH)
(74) Vertreter: Althoff, Gerhard

(57) **Zusammenfassung**

Die Erfindung betrifft eine Einrichtung zur Durchführung ophthalmologischer Eingriffe am Auge eines Lebewesens, insbesondere zum Ansaugen, Schneiden und Entfernen Teilen des Glaskörpers und/oder von Gewebeteilchen bei der Glaskörperchirurgie.

Die Einrichtung (150) umfasst ein als Handgriff ausgebildetes und mit einer Antriebseinheit (110) sowie mit einer Aspirationseinheit (115) in Verbindung stehendes Gehäuse (90) sowie eine daran angeordnete und in den Hohlraum des Glaskörpers (5) einführbare Sonde (25). Die Sonde (25) hat ein am distalen Ende mit einer Ansaugöffnung (21) versehenes Führungsrohr (20) sowie ein koaxial darin angeordnetes Innenrohr (30), welches im wesentlichen von einem im Gehäuse (90) angeordneten Antrieb (95), beispielsweise einem elektromotorischen Antrieb sowie damit wirkverbundenen und die Drehbewegung in eine Linearbewegung transformierenden Steuerelementen (65,45) derart in axialer Richtung verschiebbar ist, dass bei rotierendem Antrieb (95) sowie offener Ansaugöffnung (21) eine Ruhestellung des im Führungsrohr (20) angeordneten Innenrohres (30) erreichbar ist.

## Beschreibung

Die Erfindung bezieht sich auf eine Einrichtung zur Durchführung ophthalmologischer Eingriffe, insbesondere zum Ansaugen, Schneiden und Entfernen von Teilen des Glaskörpers und/oder Gewebeteilchen bei der Glaskörperchirurgie, bestehend aus einem Gehäuse, einem daran angeordneten und in den Hohlraum des Glaskörpers einführbaren Führungsrohr und einem koaxial darin angeordneten Innenrohr, welches von einem in dem Gehäuse angeordneten Antrieb zum Öffnen und Schliessen einer am distalen Ende des Führungsrohres vorgesehenen Ansaugöffnung in Richtung der Längsachse verschiebbar ist.

Für die Verwendung bei der Glaskörperchirurgie ist beispielsweise aus der US-A 5,833,643 eine Einrichtung bekannt, welche ein Gehäuse mit darin angeordnetem elektromotorischen Antrieb, ein am Gehäuse angeordnetes Aussenrohr, ein koaxial darin angeordnetes und an eine Aspirationsleitung angeschlossenes Innenrohr sowie eine an der Welle des elektromotorischen Antriebs befestigte und mit einer Schiebehülse wirkverbundene Taumelscheibe umfasst, mittels welcher das Innenrohr zur Erreichung einer Schneidwirkung relativ zu einer am distalen Ende des Aussenrohres vorgesehenen Ansaugöffnung oszillierend angetrieben in axialer Richtung verschiebbar ist.

Bei den an sich bekannten Einrichtungen oder Instrumenten, insbesondere zum Entfernen von Teilen des Glaskörpers oder Gewebeteilchen aus dem Glaskörperhohlraum eines Auges bewirkt der oszillierende Antrieb einerseits eine unerwünschte Vibration und andererseits können aufgrund des in Bezug auf die Ansaugöffnung zeitgleichen Öffnungsvorgangs und Schliessvorgangs folglich auch nur kleinere Gewebeteilchen erfasst, abgetrennt und abgesaugt werden. Bei längeren, zusammenhängenden Gewebeteilchen muss dieser Vorgang zum Entfernen derselben zwei oder mehrfach wiederholt werden.

Der Erfindung liegt die Aufgabe zugrunde, eine Einrichtung der eingangs genannten Art zu schaffen beziehungsweise so zu verbessern, dass ohne ohne den chirurgischen Eingriff störende Vibrationen Teile des Glaskörpers und auch längere, zusammenhängende Gewebeteilchen rasch erfasst und abgesaugt beziehungsweise erfasst sowie von dem an der Ansaugöffnung vorbeigeführten Innenrohr infolge der dabei erreichbaren Schneidwirkung abgetrennt und gleichzeitig abgesaugt werden können.

Die erfindungsgemässe Einrichtung ist gekennzeichnet durch einen die Drehbewegung des Antriebs in eine in Richtung der Längsachse orientierte Linearbewegung transformierenden Steuerkörper, von welchem das Innenrohr ausgehend von einer Ruhestellung mit offener Ansaugöffnung zum Schliessen und anschliessenden Öffnen derselben relativ dazu in axialer Richtung derart verschiebbar ist, dass bei rotierendem Antrieb sowie offener Ansaugöffnung eine Ruhestellung des im Führungsrohr angeordneten Innenrohres erreichbar ist.

Bei der erfindungsgemässen Einrichtung (High-Speed Cutter) wird die Ansaugöffnung zur Erreichung eines optimalen Ansaugvorgangs relativ lange in geöffneter Stellung gehalten während der Schliess- und Schneidvorgang für die unterschiedlich ausgebildeten und dimensionierten Teile des Glaskörpers sowie die Gewebeteilchen möglichst schnell erfolgt, wobei diese Forderung auch bei hoher Schneid- und Austragsleistung (Absaugung) gewährleistet ist.

Weitere Vorteile und Merkmale der Erfindung ergeben sich aus der nachstehenden Beschreibung in Verbindung mit dem in der Zeichnung dargestellten Ausführungsbeispiel und den einzelnen Patentansprüchen. Es zeigt:
- **Fig.1**: ein als Horizontalschnitt dargestelltes Auge sowie eine schematisch dargestellte Einrichtung mit daran angeordneter Sonde zum Ansaugen, Schneiden und Entfernen von Teilen des Glaskörpers und/oder Gewebeteilchen aus dem Glaskörperhohlraum;
- **Fig.2**: die in grösserem Massstab und teilweise im Schnitt dargestellte Einrichtung gemäss Fig.1 mit einem als Handgriff ausgebildeten Gehäuse und einem darin angeordneten Antriebssystem;
- **Fig.3**: das in grösserem Massstab und im Schnitt dargestellte Gehäuse mit dem darin angeordneten und mit der Sonde in Wirkverbindung stehenden Antriebssystem;
- **Fig.4**: ein in grösserem Massstab und im Schnitt dargestelltes Teilstück der mit einem Führungsrohr sowie einem koaxial darin angeordneten Innenrohr versehenen Sonde für die Einrichtung gemäss Fig.1;
- **Fig.5**: das Teilstück der Sonde gemäss Fig.4 mit dem relativ zu einer im Führungsrohr angeordneten Ansaugöffnung in axialer Richtung verschobenen Innenrohr;
- **Fig.6**: ein im Schnitt dargestelltes Kopfstück für das zur Aufnahme des Antriebssystems ausgebildete Gehäuse gemäss Fig.3;
- **Fig.7**: ein in Ansicht und teilweise aufgebrochen dargestelltes Zwischenstück für das zur Aufnahme des Antriebssystems ausgebildete Gehäuse gemäss Fig.3;
- **Fig.8**: ein in Ansicht und teilweise im Schnitt dargestelltes Kupplungsglied für das im Gehäuse angeordnete Antriebssystem gemäss Fig.3;
- **Fig.8A**: das in Draufsicht dargestellte Kupplungsglied gemäss Fig.8;
- **Fig.8B**: das in Seitenansicht dargestellte Kupplungsglied gemäss Fig.8A;
- **Fig.9**: einen in Ansicht dargestellten Rotationskörper für das in dem Gehäuse angeordnete Antriebssystem gemäss Fig.3;
- **Fig.9A**: den in Draufsicht dargestellten Rotationskörper gemäss Fig.9;
- **Fig.9B**: den in Seitenansicht dargestellten Rotationskörper gemäss Fig.9A;
- **Fig.10**: einen in Draufsicht dargestellten und mit einer Kurvenbahn versehenen zylindrischen Steuerkörper für das im Gehäuse angeordnete Antriebssystem gemäss Fig.3;
- **Fig.10A**: den im Schnitt dargestellten Steuerkörper gemäss Fig. 10;
- **Fig.10B**: den in Seitenansicht dargestellten Steuerkörper gemäss Fig.10A;
- **Fig.11**: die graphisch und als Abwicklung dargestellte Kurvenbahn des Steuerkörpers gemäss Fig.10; und
- **Fig.12**: den in Form mehrerer graphischer Kurven dargestellten Bewegungsablauf des von dem Steuerkörper verschiebbaren Innenrohres gemäss Fig.5.

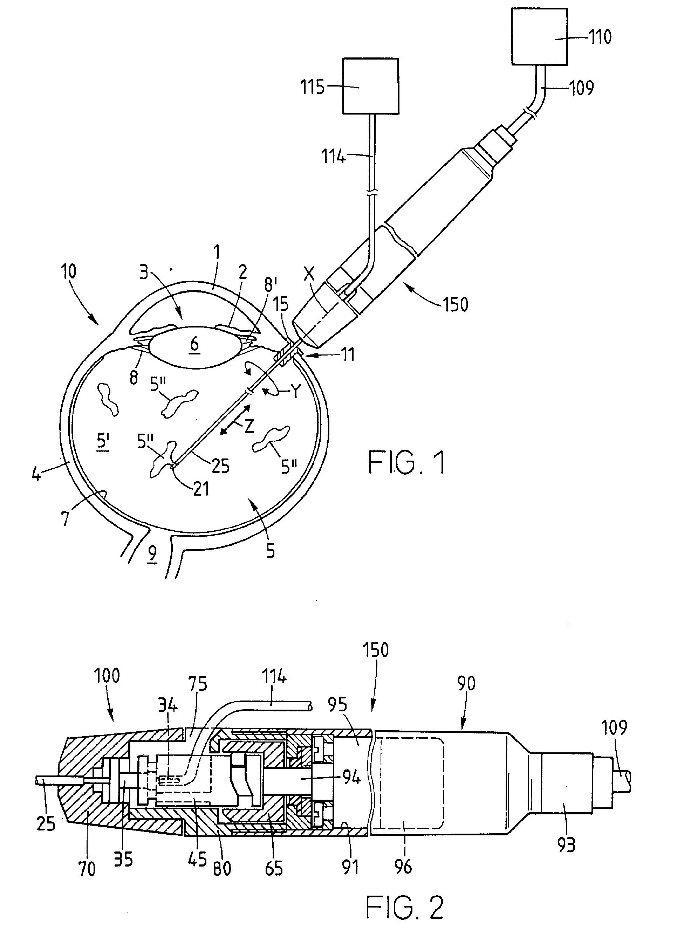

Zur Verdeutlichung der Erfindung ist in Fig.1 ein Auge 10 in grösserem Massstab und als schematischer Horizontalschnitt dargestellt und man erkennt die Hornhaut 1, die Regenbogenhaut 2, die Pupille 3, die Lederhaut 4, den Glaskörper 5 mit dem Glaskörperhohlraum 5', die Linse 6, die Netzhaut 7, die Ziliarkörper 8 mit den Zonulafasern 8' und das mit 9 bezeichnete Sehnervenbündel. Weiterhin erkennt man in Fig.1 eine schematisch dargestellte Einrichtung 150 mit daran angeordneter und in den Glaskörperhohlraum 5' eingeführter Sonde 25. Am vorderen Ende ist die längliche, röhrchenförmige Sonde 25 mit einer in Fig.1 schematisch dargestellten Ausnehmung 21 versehen und zum Ansaugen und Schneiden von Teilen 5" des Glaskörpers und/oder Gewebeteilchen sowie zum Entfernen derselben aus dem Glaskörperhohlraum 5' ausgebildet. Die Einrichtung 150 mit der daran angeordneten Sonde 25 kann manuell um die theoretische Längsachse X in Pfeilrichtung Y gedreht sowie gemäss Doppelpfeilrichtung Z in axialer Richtung verschoben werden. Zur Vermeidung von Verletzungen kann die Einrichtung 150 mit der länglichen Sonde 25 in einem Hülsenkörper 15 geführt werden, welcher etwa im Bereich der Pars plana 11 in der Lederhaut 4 angeordnet beziehungsweise eingesetzt ist.

Die Einrichtung 150 steht über eine elektrische Leitung 109 mit einer schematisch dargestellten Antriebseinheit 110 sowie über eine Aspirationsleitung 114 mit einer schematisch dargestellten Aspirationseinheit 115 in Wirkverbindung (Fig.1). Die Betätigung und Ansteuerung der Antriebseinheit 110 sowie der Aspirationseinheit 115 wird beispielsweise mittels eines nicht dargestellten Fuss-Schalters oder dergleichen erreicht.

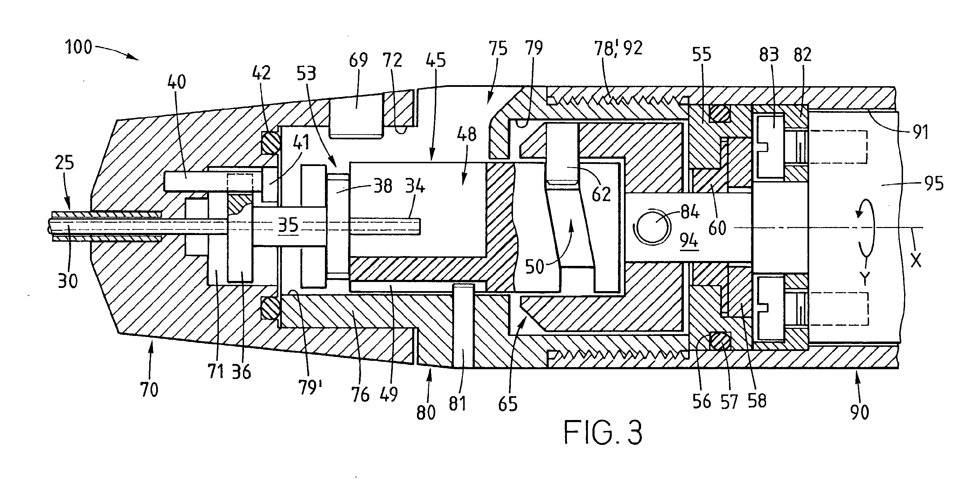

Fig.2 zeigt die in grösserem Massstab und teilweise im Schnitt dargestellte Einrichtung 150, welche ein hohlzylindrisches Gehäuse 90 in Form eines Handstücks sowie ein darin angeordnetes und in der Gesamtheit mit 100 bezeichnetes Antriebssystem umfasst. An dem hinteren Ende des Gehäuses 90 ist ein schematisch dargestelltes und zur Befestigung der elektrischen Leitung 109 ausgebildetes Verschlussteil 93 angeordnet, welches beispielsweise durch eine nicht dargestellte Schraubverbindung an dem Gehäuse 90 befestigt ist. Im Innenraum 91 des hohlzylindrischen Gehäuses 90 ist ein Antrieb, beispielsweise eine elektromotorischer Antrieb 95 sowie ein an dem einen Ende des Antriebs 95 damit wirkverbundener Drehgeber 96 angeordnet. Am anderen Ende des Antriebs 95 ist dieser mit einer um die eigene Längsachse X (Fig.3) rotierend antreibbaren Welle 94 versehen. An der Welle 94 ist ein damit wirkverbundener und etwa gabelförmig ausgebildeter Rotationskörper 65 angeordnet.

Das Antriebssystem 100 umfasst, wie in Fig.2 dargestellt, im wesentlichen den im Gehäuse 90 angeordneten Drehgeber 96 sowie den elektromotorischen Antrieb 95 mit der Welle 94, den daran angeordneten Rotationskörper 65, einen damit wirkverbundenen Steuerkörper 45 sowie ein mit der Sonde 25 in Wirkverbindung stehendes Kupplungsglied 35. Der Rotationskörper 65 und der Steuerkörper 45 sowie das Kupplungsglied 35 sind koaxial in einem an dem Gehäuse 90 durch eine Schraubverbindung befestigten Zwischenstück 80 angeordnet. Am vorderen Ende des Zwischenstücks 80 ist ein mit der Sonde 25 versehenes Kopfstück 70 angeordnet und beispielsweise durch einen sogenannten Schnellverschluss befestigt. Das Kupplungsglied 35 hat an dem einen Ende einen Stutzen 34 für den Anschluss der Aspirationsleitung 114. Die an dem Stutzen 34 angeordnete und mit nicht dargestellten Mitteln befestigte Aspirationsleitung 114 ist durch eine im Zwischenstück 80 vorgesehene Ausnehmung 75 nach aussen geführt und an die Aspirationseinheit 115 (Fig.1) angeschlossen.

Gemäss Fig.2 ist das Kopfstück 70 in axialer Richtung auf das Zwischenstück 80 aufgeschoben sowie durch eine Drehbewegung um einen Winkel von 90° relativ zu dem Zwischenstück 80 dargestellt ist, wobei die beiden Teile 70,80 in dieser Stellung durch einen Schnellverschluss miteinander verbunden sind.

In Fig.3 ist das in dem Gehäuse 90 angeordnete Antriebssystem 100 mit den einzelnen Elementen in grösserem Massstab dargestellt und man erkennt ein Teilstück des Gehäuses 90 mit dem darin angeordneten elektromotorischen Antrieb 95. Der Antrieb 95 ist beispielsweise durch einen Haltering 82 sowie Schrauben 83 im Innenraum 91 des Gehäuses 90 gehalten. Der an dem vorderen Ende der Welle 94 angeordnete und durch einen Gewindestift 84 oder dergleichen damit wirkverbundene Rotationskörper 65 wird bei entsprechender Ansteuerung des Antriebs 95 um die Längsachse X in Pfeilrichtung Y angetrieben. Zwischen dem Haltering 82 und dem in einer ersten Ausnehmung 79 des Zwischenstücks 80 angeordneten Rotationskörper 65 ist auf der Welle 94 eine Dichtung 60 angeordnet, welche durch eine Scheibe 58 und einen Distanzring 55 gegen axiales Verschieben gesichert ist. Der mit einer äusseren Ringnut 56 und darin angeordneter Dichtung 57 (O-Ring) versehene Distanzring 55 ist durch das in das Gehäuse 90 eingeschraubte Zwischenstück 80 gegen axiales Verschieben gesichert.

Weiterhin erkennt man in Fig.3 den in einer zweiten Ausnehmung 79' des Zwischenstücks 80 angeordneten und an dem einen Ende mit einer Kurvenbahn 50 versehenen zylindrischen Steuerkörper 45. In der Kurvenbahn 50 ist ein an dem gabelförmig ausgebildeten Rotationskörper 65 befestigter Bolzen 62 angeordnet, welcher bei der um die Längsachse X in Pfeilrichtung Y orientierten Drehbewegung der Welle 94 zwangsläufig in der Kurvenbahn 50 des Steuerkörpers 45 geführt ist. Der in Fig.3 teilweise im Schnitt dargestellte Steuerkörper 45 ist mit einer nut- oder schlitzförmigen Ausnehmung 48 sowie mit einer in Längsrichtung orientierten Nut 49 versehen, in welcher bei der in axialer Richtung orientierten Bewegung des Steuerkörpers 45 ein am Zwischenstück 80 befestigter Stift 81 geführt ist und somit eine Verdrehung des Steuerkörpers 45 verhindert.

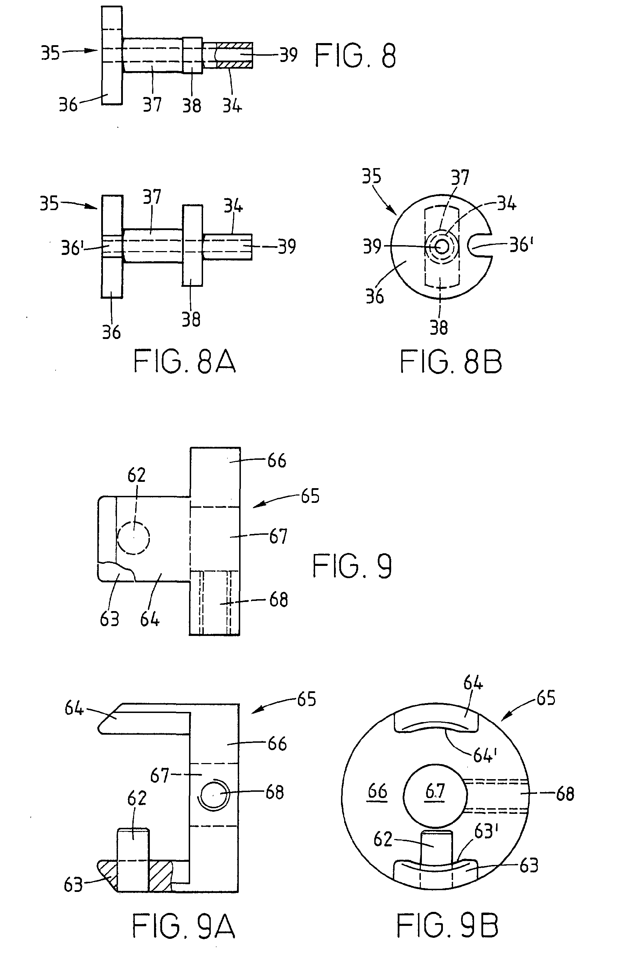

An dem anderen Ende des Steuerkörpers 45 ist das Kupplungsglied 35 angeordnet, welches mit einem Steg 38 formschlüssig in eine schlitzförmige Aussparung 53 des Steuerkörpers 45 eingreift. Das Kupplungsglied 35 ist weiterhin mit einem im Abstand zu dem Steg 38 angeordneten Flansch 36 in einer ersten Ausnehmung 71 des Kopfstücks 70 angeordnet. In dem Kopfstück 70 ist ein mit einem Anschlag 41 versehener Bolzen 40 befestigt, welcher in einer am äusseren Umfang des Flansches 36 vorgesehenen Ausnehmung 36' (Fig.8B) derart eingreift, dass das zusammen mit dem Steuerkörper 45 in axialer Richtung verschiebbare Kupplungsglied 35 gegen Verdrehung gesichert ist. Das an dem einen Ende mit dem Stutzen 34 für die Aspirationsleitung 114 versehene Kupplungsglied 35 ist an dem anderen Ende mit einem Innenrohr 30 der Sonde 25 in nicht dargestellter Weise wirkverbunden.

Das Zwischenstück 80 ist, wie in Fig.3 dargestellt, mit dem Aussengewinde 78' in das Innengewinde 92 des Gehäuses 90 eingeschraubt. In zusammengebautem Zustand ist das Kopfstück 70 in Bezug auf das Zwischenstück 80 beispielsweise durch einen an der Stirnseite (nicht bezeichnet) des in eine Ausnehmung 72 eingeschobenen zylindrischen Teilstücks 76 anliegenden O-Ring 42 abgedichtet.

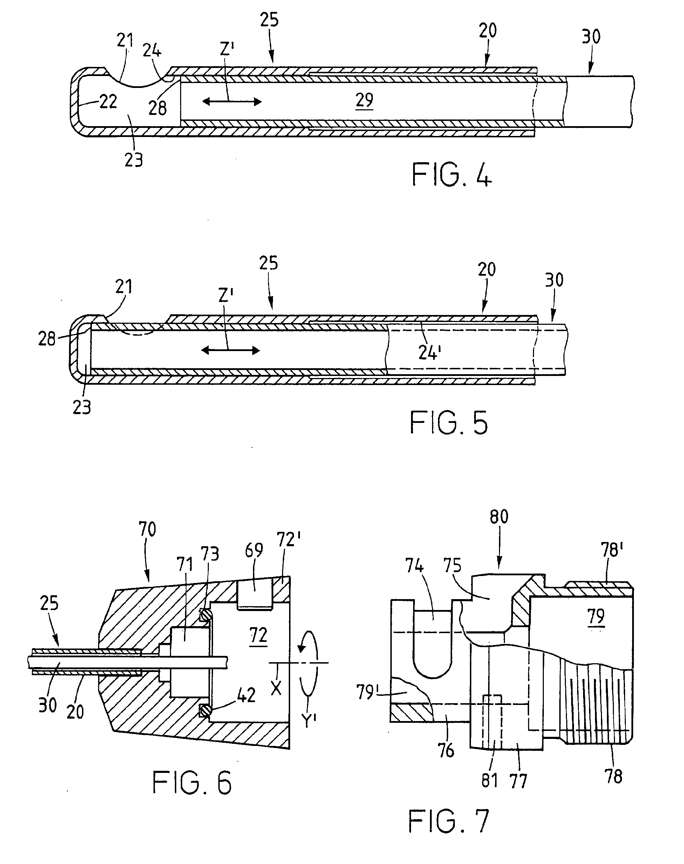

Weiterhin erkennt man in Fig.3 das mit der länglichen rohrförmigen Sonde 25 versehene Kopfstück 70, welches beispielsweise durch einen daran angeordneten bolzenförmigen Rastnokken 69 in Form eines Bajonett-Verschlusses oder dergleichen auswechselbar an dem Zwischenstück 80 befestigt ist. In Fig.3 ist das Kopfstück 70 mit einzelnen Funktionselementen in der auf das Zwischenstück 80 aufgeschobenen Stellung dargestellt. Durch eine um die Längsachse X orientierte Drehbewegung des Kopfstücks 70 mit einem Winkel von 90° wird der Rastnocken 69 schnappartig mit einer am Zwischenstück 80 vorgesehenen Ausnehmung 74 (Fig.7) in Eingriff gebracht.

In Fig.4 ist das vordere Ende der Sonde 25 in grösserem Massstab und im Schnitt dargestellt, welche ein äusseres Führungsrohr 20 sowie das koaxial darin angeordnete und gemäss Doppelpfeilrichtung Z' verschiebbare Innenrohr 30 umfasst. Am vorderen Ende ist das äussere Führungsrohr 20 durch eine Stirnwand 22 verschlossen. Im Abstand zu der Stirnwand 22 ist in dem Führungsrohr 20 eine Ausnehmung 21 angeordnet, welche nachstehend als Ansaugöffnung 21 bezeichnet wird. Zur Führung und besseren koaxialen Zentrierung des Innenrohres 30 ist das Führungsrohr 20 im Bereich der Ausnehmung 21 beispielsweise mit einem in axialer Richtung orientierten und abgesetzt ausgebildeten Teilstück 24 und 24' versehen. Bei der in Fig.4 dargestellten Position (Ruhestellung) ist das Innenrohr 30 in Bezug auf die Stirnwand 22 des Führungsrohres 20 zurückgezogen dargestellt, so dass die Ausnehmung 21 zum Ansaugen der Gewebeteilchen 5" (Fig.1) offen ist.

Zur Verdeutlichung der Dimension der in den Hohlraum 5' des Glaskörpers 5 einführbaren Sonde 25 wird darauf hingewiesen, dass bei einem bevorzugten Ausführungsbeispiel das Führungsrohr 20 einen äusseren Durchmesser von etwa 0,91 mm und das koaxial darin verschiebbare Innenrohr 30 einen äusseren Durchmesser von etwa 0,63 mm hat. Die am distalen Ende des Führungsrohres 20 vorgesehene Ansaugöffnung 21 hat eine in axialer Richtung orientierte Länge von etwa 0,7 mm.

Bei der in Fig.5 dargestellten Stellung ist das Innenrohr 30 mit der vorderen in nicht näher dargestellter Weise als Schneidkante ausgebildeten Stirnseite 28 in Richtung der Stirnwand 22 verschoben, so dass das infolge der Aspirationswirkung durch die Ansaugöffnung 21 in den Innenraum 23 des Führungsrohres 20 gezogene Gewebeteilchen 5" (Fig.1) von der Schneid- oder Stirnseite 28 abgetrennt und anschliessend durch den in axialer Richtung orientierten Kanal 29 des Innenrohres 30 sowie über die Leitung 114 abgesaugt wird.

In Fig.6 ist das Kopfstück 70 sowie die darin angeordnete Sonde 25 im Schnitt dargestellt und man erkennt ein Teilstück des im Kopfstück 70 eingesteckten Führungsrohres 20 sowie das koaxial darin angeordnete Innenrohr 30. Das Führungsrohr 20 ist beispielsweise durch eine Klebverbindung oder dergleichen in dem Kopfstück 70 befestigt. Das Kopfstück 70 hat eine erste zylindrische Ausnehmung 71 sowie die zweite zylindrische Ausnehmung 72. An der nicht bezeichneten Stirnwand der zweiten Ausnehmung 72 ist eine Ringnut 73 für die Dichtung 42 (Fig.3) vorgesehen. In der zirkulären Wand 72' des Kopfstücks 70 ist weiterhin ein teilweise in die zweite Ausnehmung 72 ragender Rastnocken 69 angeordnet und mit nicht dargestellten Mitteln befestigt.

Fig.7 zeigt das in Ansicht und teilweise aufgebrochen dargestellte Zwischenstück 80, welches ein erstes zylindrisches Teilstück 76, ein zweites zylindrisches Teilstück 77 und ein daran angeformtes und mit dem Aussengewinde 78' versehenes drittes Teilstück 78 umfasst. Das Zwischenstück 80 hat eine in axialer Richtung orientierte erste zylindrische Ausnehmung 79 sowie eine abgesetzt dazu ausgebildete zweite zylindrische Ausnehmung 79'. Weiterhin erkennt man in Fig.7 die in axialer Richtung orientierte und etwa spaltförmig ausgebildete Ausnehmung 75 für die Aspirationsleitung 114 (Fig.2). In der nicht bezeichneten Wand des zweiten zylindrischen Teilstücks 77 ist ein in die zweite Ausnehmung 79' ragender Stift 81 eingesetzt und befestigt. Mit dem Stift 81 ist der in die zweite Ausnehmung 79' des Zwischenstücks 80 einschiebbare Steuerkörper 45 (Fig.3) in nicht näher dargestellter Weise gegen Verdrehung gesichert.

Das Zwischenstück 80 ist an dem ersten zylindrischen Teilstück 76 mit einer teilweise in Umfangsrichtung orientierten Nut 74 versehen (Fig.7). Die Nut 74 ist so ausgebildet, dass das auf das erste zylindrische Teilstück 76 aufschiebbare Kopfstück 70 mit dem Rastnocken 69 in die Nut 74 eingreift und durch die um die gemeinsame Längsachse X in Pfeilrichtung Y' orientierte Drehbewegung mit einem Winkel von etwa 90° durch den vorstehend erwähnten Bajonett-Verschlusses mit dem Zwischenstück 80 verbunden ist. Das mit der röhrchenförmigen Sonde 25 versehene Kopfstück 70 kann durch den Bajonett-Verschluss zum Reinigen (Desinfizierung) der Sonde 25 beziehungsweise zum Auswechseln des kompletten Kopfstücks 70 schnell und ohne zusätzliche Hilfsmittel von dem Zwischenstück 80 beziehungsweise von dem Gehäuse 90 entfernt werden.

Fig.8 zeigt das in Ansicht dargestellte Kupplungsglied 35, welches einen von einer Durchgangsbohrung 39 durchdrungenen Zylinderkern 37, den am vorderen Ende desselben angeordneten Flansch 36 sowie den in axialer Richtung im Abstand dazu angeordneten Steg 38 aufweist. Der am hinteren Ende teilweise im Schnitt dargestellte Zylinderkern 37 ist in diesem Bereich als abgesetzter zylindrischer Stutzen 34 zum Aufschieben und Anschliessen der Aspirationsleitung 114 (Fig.2) ausgebildet.

In Fig.8A ist das Kupplungsglied 35 in Draufsicht und in Fig.8B in Seitenansicht dargestellt und man erkennt den am äusseren Umfang mit einer Ausnehmung 36' versehenen und als kreisförmige Scheibe ausgebildeten Flansch 36, den in axialer Richtung von der Durchgangsbohrung 39 durchdrungenen Zylinderkern 37 mit dem Stutzen 34 sowie den vertikal und quer zur Längsrichtung des Zylinderkerns 37 angeordneten Steg 38 (Fig.8B).

Fig.9 zeigt den in Ansicht dargestellten und etwa gabelförmig ausgebildeten Rotationskörper 65, welcher ein scheibenförmig ausgebildetes Seitenteil 66 sowie zwei daran angeformte und im Abstand zueinander angeordnete Gabelarme 63 und 64 aufweist. An dem einen Gabelarm 63 ist der Bolzen 62 angeordnet und mit nicht dargestellten Mitteln befestigt. Das Seitenteil 66 ist mit einer Durchgangsbohrung 67 und einer quer dazu angeordneten Gewindebohrung 68 versehen, wobei in zusammengebautem Zustand (Fig.3) die Welle 94 des elektromotorischen Antriebs 95 in der Durchgangsbohrung 67 angeordnet und durch den Gewindestift 84 befestigt ist.

In Fig.9A ist der Rotationskörper 65 in Draufsicht und in Fig.9B in Seitenansicht dargestellt und man erkennt das mit der Gewindebohrung 68 und der Durchgangsbohrung 67 versehene Seitenteil 66. An dem Seitenteil 66 sind die beiden parallel zueinander angeordneten Gabelarme 63 und 64 angeordnet, wobei der eine Gabelarm 63 mit dem Bolzen 62 versehen ist. Die beiden an dem Seitenteil 66 gegenüberliegend zueinander angeordneten Gabelarme 63 und 64 sind, wie in Fig.9B dargestellt, an den einander zugewandten Innenseiten 63' und 64' entsprechend dem zylindrischen Steuerkörper 45 etwa kreisbogenförmig ausgebildet.

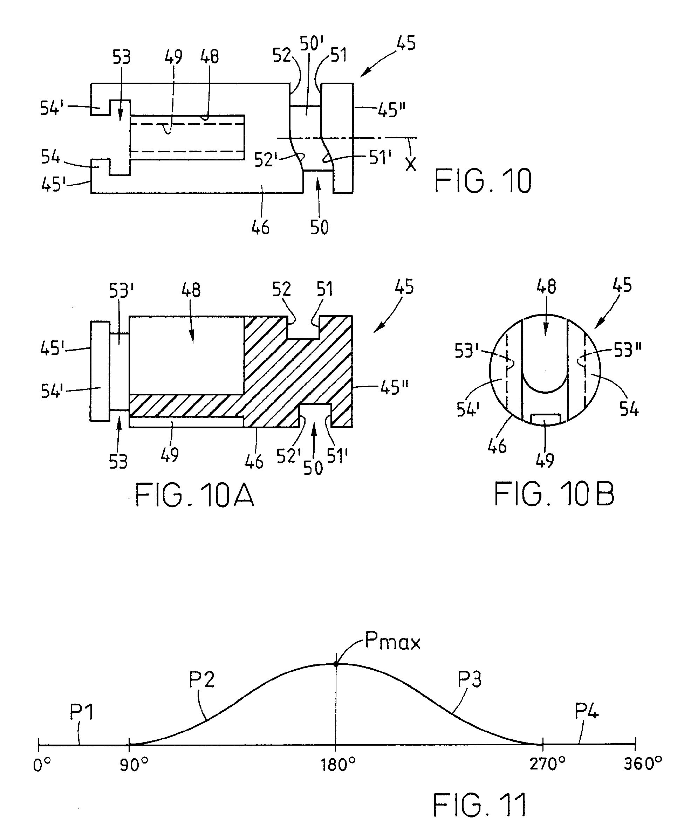

Fig.10 zeigt den in Draufsicht dargestellten zylindrischen Steuerkörper 45, welcher an dem einen Ende im Abstand zu der Stirnwand 45' mit der quer zur Längsachse X angeordneten Aussparung 53 und an dem anderen Ende im Abstand zu der Rückwand 45" mit der Kurvenbahn 50 versehen ist. Ausgehend von der Stirnwand 45' ist der Steuerkörper 45 weiterhin mit einer in axialer Richtung orientierten länglichen Ausnehmung 48 und korrespondierend dazu am äusseren Umfang mit einer Längsnut 49 versehen. Die in dem Steuerkörper 45 angeordnete und mit einem Zylinderkern 50' versehene Kurvenbahn 50 ist etwa nut- oder schlitzförmig ausgebildet. Die Kurvenbahn 50 setzt sich in Umfangsrichtung gesehen im wesentlichen aus zwei zusammenhängende, kreisbogenförmige Teilstücke (nicht bezeichnet) zusammen, wobei das eine Teilstück die beiden orthogonal zur Längsachse X orientieren Seitenwände 51 und 52 und das andere Teilstück die beiden schräg dazu in Richtung der Rückwand 45" des Steuerkörpers 45 geneigt ausgebildeten Seitenwände 51' und 52' aufweist.

Die Seitenwände 51,52 sowie 51',52' der in Umfangsrichtung an dem Steuerkörper 45 angeordneten Kurvenbahn 50 sind parallel im Abstand zueinander angeordnet. Der nicht bezeichnete Abstand zwischen den Seitenwänden der Kurvenbahn 50 ist so ausgebildet, dass der Bolzen 62 des um die Längsachse X rotierend angetriebenen Rotationskörpers 65 (Fig.3) exakt geführt ist und infolge dessen die in axialer Richtung orientierte Bewegung des Steuerkörpers 45 zusammen mit dem Kupplungsglied 35 und dem Innenrohr 30 erreicht wird.

In Fig.10A ist der Steuerkörper 45 als Längsschnitt und in Fig.10B in Seitenansicht dargestellt und man erkennt das an dem einen Ende durch die Stirnwand 45' und an dem anderen Ende durch die Rückwand 45" begrenzte zylindrische Teilstück 46. Im Abstand zu der Rückwand 45" ist an dem Steuerkörper 45 die mit den Seitenwänden 51,51' und 52,52' versehene zirkuläre Kurvenbahn 50 angeordnet. In dem zylindrischen Teilstück 46 ist die in axialer Richtung orientierte und bis zur Stirnwand 45' reichende Ausnehmung 48 angeordnet. Die Stirnwand 45' ist aufgrund der in axialer Richtung orientierten Ausnehmung 48 in zwei segmentförmige Wandteile 54 und 54' unterteilt. Am äusseren Umfang ist weiterhin die in axialer Richtung am zylindrischen Teilstück 46 angeordnete Längsnut 49 vorgesehen. Im Abstand zu der Stirnwand 45' ist die quer zur Längsrichtung angeordnete Aussparung 53 vorgesehen, welche durch die beiden Innenwände 53' und 53" begrenzt ist (Fig.10B). Weiterhin erkennt man in Fig.10B die Ausnehmung 48 sowie die beiden segmentförmigen Wandteile 54 und 54'.

Die Funktion der Einrichtung 150 in Verbindung mit dem im Gehäuse 90 angeordneten elektromotorischen Antrieb 95 und Drehgeber 96 sowie der Antriebseinheit 110 und der Aspirationseinheit 115 wird nachstehend beschrieben:

Für eine einwandfreie Ansaug- und Schneidfunktion ist eine exakte Einstellung und Fixierung des in axialer Richtung verschiebbaren Innenrohres 30 in Bezug auf die im Führungsrohr 20 angeordneten Ansaugöffnung 21 erforderlich. Zur Erreichung dieser Voraussetzungen werden beispielsweise von dem Drehgeber 96 in Abhängigkeit der Drehbewegung zwei phasenverschobene elektrische Signale erzeugt. Die elektrischen Signale werden einerseits zur Ermittlung und Bestimmung der momentanen Drehrichtung des Antriebs 95 herangezogen und andererseits zur Ermittlung und Bestimmung der momentanen Position (erster Referenzpunkt) des Innenrohres 30 in Bezug auf die am distalen Ende des Führungsrohres 20 vorgesehene Ansaugöffnung 21 verwendet.

Zur Ermittlung des ersten Referenzpunktes (Fig.4) wird zunächst bei eingeschalteter Aspirationseinheit 115 der elektromotorische Antrieb 95 solange gedreht und dabei das Innenrohr 30 axial in Richtung der Ansaugöffnung 21 verschoben bis das Vakuum ansteigt und bei Erreichung eines zweiten Referenzpunktes (Fig.5) sowie völlig geschlossener Ansaugöffnung 21 (Fig.5) der absolute Wert des Vakuums erreicht ist. Die beiden in axialer Richtung beabstandeten Referenzpunkte sind durch die Drehung der einzelnen Funktionselemente 94,65 und 45 um einen Drehwinkel von 180° in axialer Richtung relativ im Abstand zueinander angeordnet. Mit dem ersten Referenzpunkt (nicht näher bezeichnet) wird die sogenannte Ruhestellung bei völlig geöffneter Ansaugöffnung 21 (Fig.4) und mit dem zweiten Referenzpunkt (nicht näher bezeichnet) die Schliess-Stellung der Ansaugöffnung 21 (Fig.5) definiert und fixiert.

In Fig.11 ist die in dem Steuerkörper 45 angeordnete Kurvenbahn 50 als Abwicklung dargestellt. Die Kurvenbahn 50 ist vorzugsweise so ausgebildet, dass bei einer um die Längsachse X orientierten Drehbewegung der Welle 94 zusammen mit dem Rotationskörper 65 um einen Winkel von 0° bis 360° die nachstehend im einzelnen aufgeführten Drehphasen P1 bis P4 beziehungsweise daraus resultierende und in axialer Richtung orientierten Bewegungsabläufe des mit dem Rotationskörper 65 wirkverbundenen Steuerkörpers 45 erreicht werden, wobei
**a)** in der ersten Drehphase P1 um den Winkel von etwa 0° bis 90° der Steuerkörper 45 von dem in der Kurvenbahn 50 zwangsläufig geführten Bolzen 62 des gabelförmigen Rotationskörpers 65 nicht aktiviert wird und keine axiale Verschiebung des Steuerkörpers 45 erfolgt, so dass das Innenrohr 30 stationär im Abstand (Ruhestellung) zu der am distalen Ende des Aussenrohres 20 vorgesehenen Ansaugöffnung 21 angeordnet ist;
**b)** in der zweiten Drehphase P2 um den Winkel von 90° bis 180° der Steuerkörper 45 von dem in der Kurvenbahn 50 zwangsläufig geführten Bolzen 62 aktiviert und infolge dessen eine axiale Verschiebung des Steuerkörpers 45 mit dem Innenrohr 30 bis in eine die Ansaugöffnung 21 verschliessende und in Fig.11 mit Pₘₐₓ bezeichnete Endstellung erreicht wird;
**c)** in der dritten Drehphase P3 um den Winkel von 180° bis 270° der Steuerkörper 45 mit dem Innenrohr 30 in axialer Richtung bis in eine die Ansaugöffnung (21) wieder öffnende Ausgangsstellung zurückgeführt wird; und
**d)** in der vierten Drehphase P4 um den Winkel von 270° bis 360° der Steuerkörper 45 nicht aktiviert wird und das Innenrohr (30) stationär im Abstand zu der Ansaugöffnung (21) angeordnet ist.

Bei den vorstehend beschriebenen Drehphasen P1 bis P4 wird die Rotations- oder Drehbewegung der Welle 94 von dem Bolzen 62 des Rotationskörpers 65 derart auf den Steuerkörper 45 sowie auf das damit wirkverbundene Innenrohr 30 übertragen, dass dieses während der Drehphase P1 um den Winkel von 0° bis 90° in der die Ansaugöffnung 21 offenhaltenden Ruhestellung stehen bleibt. Bei der anschliessenden Drehphase P2 des Rotationskörpers 65 um den Winkel von 90° bis 180° wird der Steuerkörper 45 mit dem Innenrohr 30 zum Schliessen und bei der Drehphase P3 um den Winkel von 180° bis 270° zum Öffnen der Ansaugöffnung 21 entsprechend in axialer Richtung verschoben. Die nachfolgende Drehphase P4 um den Winkel von 270° bis 360° ist analog der Drehphase P1, bei welcher der Steuerkörper 45 mit dem Innenrohr 30 in der Ruhestellung stehen bleibt und somit die Ansaugöffnung 21 geöffnet ist. Bei den vorstehenden Drehphasen P1 bis P4 ist die Zeitgrösse der Drehphasen P1 und P4 (Ruhestellung) zum Ansaugen der Gewebeteilchen mindestens gleich gross wie die gesamte Zeitgrösse der Drehphasen P2 und P3 zum Schliessen und wieder Öffnen der Ansaugöffnung 21 in dem Aussenrohr 20.

An dieser Stelle wird darauf hingewiesen, dass beim Abschalten der gesamten Einrichtung 150 (Fig.2) der elektromotorische Antrieb 95 von dem Drehgeber 96 immer zu dem Zeitpunkt stoppt, wenn das Innenrohr 30 in Bezug auf die Ansaugöffnung 21 in der Offenstellung (Fig.4) ist. Hiermit wird gewährleistet, dass jeweils beim Starten der Einrichtung 150 zunächst das einzelne Gewebeteilchen 5" (Fig.1) angesaugt und anschliessend bei der axialen Relativbewegung des Innenrohres 30 in Bezug auf die Ansaugöffnung 21 des Aussenrohres 20 abgetrennt wird.

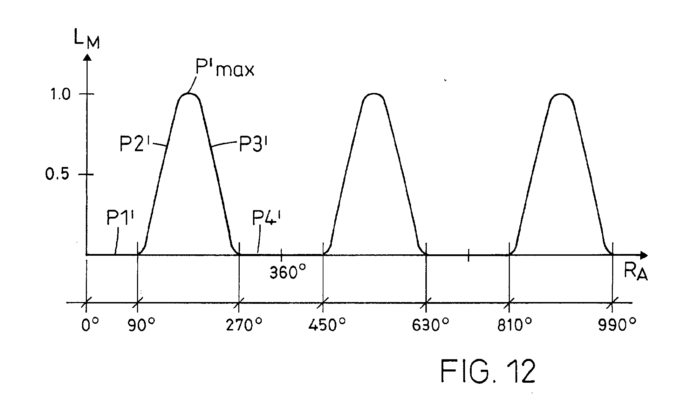

Fig.12 zeigt die in Verbindung mit einem Koordinatensystem als graphische Kurven-Addition dargestellten Bewegungsabläufe sowie daraus resultierenden Bewegungen. Bei dem Koordinatensystem sind ausgehend von dem mit P_{I} (point of intercept) bezeichneten Schnittpunkt auf der mit R_{A} (rotation angle) bezeichneten Abszissenachse eine beliebig gewählte Anzahl der Umdrehungen dargestellt. Auf der mit L_{M} (lifting motion) bezeichneten Ordinatenachse ist die infolge der Kurvenbahn 50 in axialer Richtung orientierte Bewegung des Steuerkörpers 45 zum "Öffnen" und anschliessenden "Schliessen" der im Führungsrohr 20 vorgesehenen Ansaugöffnung 21 angegeben. Die einzelnen Drehphasen P1' bis P4' der Welle 94 und der dadurch bewirkten und in axialer Richtung orientierten Bewegungsabläufe des Steuerkörpers 45 und des Kupplungsgliedes 35 zusammen mit dem Innenrohr 30 werden nachstehend beschrieben.

Wie in Fig.12 schematisch dargestellt, erfolgt ausgehend von dem Schnittpunkt P_{I} bei der um den Drehwinkel R_{A} von 0° bis 90° bewirkten ersten Drehphase P1' keine Bewegung L_{M} und somit auch keine axiale Verschiebung der einzelnen Elemente 45,35 und 30, so dass in dieser Phase, wie in Fig.4 dargestellt, die in dem feststehenden Führungsrohr 20 angeordnete Ansaugöffnung 21 offen ist (erster Referenzpunkt).

In der nachfolgenden zweiten Drehphase P2' um den Drehwinkel R_{A} von 90° bis 180° erfolgt eine erste Bewegung L_{M} und somit eine axiale Verschiebung der Elemente 45,35 und 30 bis in die mit P'ₘₐₓ bezeichnete Endstellung, so dass in dieser Phase, wie in Fig.5 dargestellt, die in dem Führungsrohr 20 angeordnete Ansaugöffnung 21 mittels des in axialer Richtung in die Endstellung verschobenen Innenrohres 30 geschlossen ist (zweiter Referenzpunkt).

Bei der anschliessenden dritten Drehphase P3' um den Drehwinkel R_{A} von 180° bis 270° erfolgt ausgehend von der Endstellung P'ₘₐₓ eine Umkehrung der ersten Bewegung L_{M} und infolge davon eine axiale Verschiebung der Elemente 45,35 und 30, so dass das Innenrohr 30 sukzessive ausser Eingriff der Ansaugöffnung 21 gelangt und, wie in Fig.4 dargestellt, wieder geöffnet wird.

Bei der nachfolgenden vierten Drehphase P4' um den Drehwinkel R_{A} von 270° bis 360° beziehungsweise bei der Drehbewegung der Antriebswelle 94 zusammen mit dem Rotationskörper 65 um den Drehwinkel R_{A} von 270° bis 450° ist die Ansaugöffnung 21 geöffnet.

Die erfindungsgemässe Einrichtung 150 gewährleistet bei relativ hohen Schnittzahlen und optimaler Abtrags- sowie Absaugleistung eine weitgehend vibrationslose Handhabung. Eine entsprechend präzise Steuerung des elektromotorischen Antriebes 95 gewährleistet neben der hohen Schnittzahlen auch eine relativ geringe Anzahl von Einzelschnitten, so dass die Verwendung auch an sogenannten Problemstellen möglich ist.

An dieser Stelle wird darauf hingewiesen, dass die Erfindung nicht auf das vorstehend beschriebene Ausführungsbeispiel mit den einzelnen Elementen beschränkt ist, so dass weitere zweckmässige Ausgestaltungen ohne dabei den Grundgedanken in Form der beschriebenen speziellen Funktionsabläufe der Erfindung zu verlassen, ebenfalls möglich sind.

## Patentansprüche

1. Einrichtung zur Durchführung ophthalmologischer Eingriffe, insbesondere zum Ansaugen, Schneiden und Entfernen von Teilen des Glaskörpers und/oder Gewebeteilchen bei der Glaskörperchirurgie, bestehend aus einem Gehäuse (90), einem daran angeordneten und in den Hohlraum des Glaskörpers (5) einführbaren Führungsrohr (20) und einem koaxial darin angeordneten Innenrohr (30), welches von einem in dem Gehäuse (90) angeordneten Antrieb (95) zum Öffnen und Schliessen einer am distalen Ende des Führungsrohres (20) vorgesehenen Ansaugöffnung (21) in Richtung der Längsachse (X) verschiebbar ist, **gekennzeichnet durch** einen die Drehbewegung des Antriebs (95) in eine in Richtung der Längsachse (X) orientierte Linearbewegung transformierenden Steuerkörper (45), von welchem das Innenrohr (30) ausgehend von einer Ruhestellung mit offener Ansaugöffnung (21) zum Schliessen und anschliessenden Öffnen derselben relativ dazu in axialer Richtung derart verschiebbar ist, dass bei rotierendem Antrieb (95) sowie offener Ansaugöffnung (21) eine Ruhestellung des im Führungsrohr (20) angeordneten Innenrohres (30) erreichbar ist.

2. Einrichtung nach Anspruch 1, **gekennzeichnet durch** einen an dem einen Ende mit der Welle (94) des Antriebs (95) und an dem anderen Ende mit dem Steuerkörper (45) wirkverbundenen Rotationskörper (65), wobei der Rotationskörper (65) mit einem daran angeordneten Bolzen (62) in einer am äusseren Umfang des zylindrischen Steuerkörpers (45) angeordneten Kurvenbahn (50) derart geführt ist, dass der gegen Verdrehung gesicherte Steuerkörper (45) zusammen mit dem über ein Kupplungsstück (35) damit wirkverbundenen Innenrohr (30) in Abhängigkeit der Kurvenbahn (50) relativ zu der Ansaugöffnung (21) des Führungsrohres (20) in axialer Richtung verschiebbar ist.

3. Einrichtung nach Anspruch 1, **gekennzeichnet durch** eine zeitlich begrenzte Ruhestellung zum Ansaugen des Glaskörpers und/oder der Gewebeteilchen, wobei die Zeitgrösse der Ruhestellung des in axialer Richtung im Führungsrohr (20) relativ zu der Ansaugöffnung (21) verschiebbaren Innenrohres (30) mindestens gleich gross wie die Addition der beiden Zeitgrössen zum Schliessen und anschliessenden Öffnen der Ansaugöffnung (21) ist.

4. Einrichtung nach Anspruch 2, **dadurch gekennzeichnet, dass** bei jeder um die Längsachse (X) orientierten Umdrehung des Rotationskörpers (65) um einen Drehwinkel von 0° bis 360° der Steuerkörper (45) zusammen mit dem Innenrohr (30) ausgehend von der Ruhestellung mindestens einmal zum Schliessen und wieder zum Öffnen der Ansaugöffnung (21) relativ dazu in axialer Richtung verschiebbar ist.

5. Einrichtung nach Anspruch 4, **dadurch gekennzeichnet, dass** bei jeder Umdrehung des Rotationskörpers (65) um den Drehwinkel von 0° bis 360° der damit wirkverbundene Steuerkörper (45) ausschliesslich bei der Umdrehung im Bereich des Drehwinkels von etwa 90° bis 270° zusammen mit dem Innenrohr (30) in axialer Richtung verschiebbar ist.

6. Einrichtung nach Anspruch 2, **dadurch gekennzeichnet, dass** der Steuerkörper (45) ausgehend von der dem Rotationskörper (65) gegenüberliegenden Stirnseite (45') eine in axialer Richtung orientierte Ausnehmung (48) zur Aufnahme des mit dem Innenrohr (30) wirkverbundenen Kupplungsgliedes (35) aufweist.

7. Einrichtung nach Anspruch 6, **dadurch gekennzeichnet, dass** der Steuerkörper (45) im Abstand zu der Stirnseite (45') eine quer zu der in Längsrichtung orientierten Ausnehmung (48) angeordnete Aussparung (53) aufweist, in welche ein an dem Kupplungsglied (35) angeordneter Steg (38) formschlüssig einsetzbar ist.

8. Einrichtung nach Anspruch 6, **dadurch gekennzeichnet, dass** das Kupplungsglied (35) mit einem in die Ausnehmung (48) des Steuerkörpers (45) ragenden und zum Anschliessen einer Aspirationsleitung (114) ausgebildeten Stutzen (34) versehen ist.

9. Einrichtung nach Anspruch 2, **dadurch gekennzeichnet, dass** der Rotationskörper (65) auf der dem Steuerkörper (45) zugewandten Seite zwei gegenüberliegend und im Abstand zueinander angeordnete sowie in Richtung des Steuerkörpers (45) orientierte Gabelarme (63,64) aufweist, wobei an dem einen Gabelarm (63) der in der zirkulären Kurvenbahn (50) des Steuerkörpers (45) geführte Bolzen (62) angeordnet ist.

10. Einrichtung nach Anspruch 9, **dadurch gekennzeichnet, dass** die beiden in axialer Richtung über den zylindrischen Steuerkörper (45) greifenden Gabelarme (63,64) an den einander zugewandten Innenseiten (63',64') analog dem Aussendurchmesser des Steuerkörpers (45) kreisbogenförmig ausgebildet sind.

11. Einrichtung nach Anspruch 2, **dadurch gekennzeichnet, dass** die an dem Steuerkörper (45) angeordnete Kurvenbahn (50) einen in Bezug auf den Aussendurchmesser kleineren Zylinderkern (50') sowie zwei parallel in Umfangsrichtung orientierte und mit zwei ersten halbkreisförmigen Teilstücken (51,52) und mit zwei kurvenförmig daran angeformten zweiten halbkreisförmigen Teilstücken (51',52') versehene Seitenwände aufweist.

12. Einrichtung nach Anspruch 11, **dadurch gekennzeichnet, dass** die beiden ersten halbkreisförmigen Teilstücke (51,52) orthogonal zu der Längsachse (X) und die beiden zweiten halbkreisförmigen Teilstücke (51',52') etwa kurvenförmig schräg in Richtung der Rückwand (45") des Steuerkörpers (45) geneigt an dem Zylinderkern (50') angeformt sind, wobei die einzelnen Teilstücke (51,52;51',52') die in Umfangsrichtung orientierten Seitenwände für die Kurvenbahn (50) bilden.

13. Einrichtung nach Anspruch 2, **gekennzeichnet durch** ein an dem Gehäuse (90) angeordnetes Zwischenstück (80) sowie ein daran angeordnetes und mit dem Führungsrohr (20) sowie dem koaxial darin angeordneten Innenrohr (30) versehenes Kopfstück (70), wobei das Kopfstück (70) in axialer Richtung auf das Zwischenstück (80) aufschiebbar und **durch** eine relativ dazu um die Längsachse (X) orientierte Drehbewegung in Form eines Schnellverschlusses daran befestigt ist.

14. Einrichtung nach Anspruch 13, **dadurch gekennzeichnet, dass** das Kopfstück (70) eine hohlzylindrische Ausnehmung (71) aufweist, in welcher das an dem einen Ende mit dem Steuerkörper (45) wirkverbundene und in axialer Richtung verschiebbare Kupplungsstück (35) mit einem an dem anderen Ende angeformten Flansch (36) gegen Verdrehung gesichert ist.

15. Einrichtung nach Anspruch 14, **dadurch gekennzeichnet, dass** das Kopfstück (70) zusammen mit dem Kupplungsstück (35) als eine Baueinheit in axialer Richtung auf das Zwischenstück (80) derart aufschiebbar ist, dass durch eine um die Längsachse (X) orientierte Drehbewegung das Kopfstück (70) mit dem Zwischenstück (80) und das Kupplungsstück (35) über den Steg (38) mit dem Steuerkörper (45) wirkverbunden ist.

16. Einrichtung nach Anspruch 13, **dadurch gekennzeichnet, dass** das Zwischenstück (80) zur Aufnahme des mit der Welle (94) des Antriebs (95), beispielsweise eines elektromotorischen Antriebs wirkverbundenen Rotationskörpers (65) sowie des damit wirkverbundenen Steuerkörpers (45) ausgebildet ist, wobei der mit einer in axialer Richtung orientierten Nut (49) versehene Steuerkörper (45) durch einen an dem Zwischenstück (80) angeordneten und in die Nut (49) eingreifenden Bolzen (81) gegen Verdrehung gesichert ist.

17. Einrichtung nach Anspruch 13, **dadurch gekennzeichnet, dass** das Zwischenstück (80) eine in axialer Richtung orientierte und spaltförmig ausgebildete Ausnehmung (75) aufweist, durch welche die an dem Stutzen (34) des Kupplungsgliedes (35) angeordnete Aspirationsleitung (114) aus der kammerförmigen Ausnehmung (79') des Zwischenstücks (80) herausführbar ist.

18. Einrichtung nach Anspruch 13, **dadurch gekennzeichnet, dass** das in axialer Richtung auf das Zwischenstück (80) aufschiebbare Kopfstück (70) mit einem Rastnocken (69) versehen ist, welcher in aufgeschobener Endstellung durch eine Drehbewegung um einen Winkel von etwa 90° in einer am äusseren Umfang des Zwischenstücks (80) angeordneten Nut (74) schnappartig einrastend angeordnet ist.

19. Einrichtung nach Anspruch 1, **dadurch gekennzeichnet, dass** die um die Längsachse (X) orientierte Umdrehung des von dem Antieb (95) rotierend angetriebenen Rotationskörpers (65) derart in einzelne Drehphasen (P1 bis P4) unterteilt ist, dass
**a)** bei einer ersten Drehphase (P1) um den Winkel von etwa 0° bis 90° der Steuerkörper (45) in einer Ausgangsstellung stehen bleibt und das damit wirkverbundene Innenrohr (30) stationär im Abstand zu der am distalen Ende des Aussenrohres (20) vorgesehenen Ansaugöffnung (21) angeordnet ist;
**b)** bei der zweiten Drehphase (P2) um den Winkel von etwa 90° bis 180° der Steuerkörper (45) mit dem Innenrohr (30) in axialer Richtung bis in eine die Ansaugöffnung (21) verschliessende Endstellung verschiebbar ist;
**c)** bei der dritten Drehphase (P3) um den Winkel von etwa 180° bis 270° der Steuerkörper (45) mit dem Innenrohr (30) in axialer Richtung bis in die die Ansaugöffnung (21) wieder öffnende Ausgangsstellung zurückführbar ist;
**d)** bei der vierten Drehphase (P4) um den Winkel von etwa 270° bis 360° der Steuerkörper (45) in der Ausgangsstellung stehen bleibt, in welcher das Innenrohr (30) im Abstand zu der Ansaugöffnung (21) des Aussenrohres (20) angeordnet ist.

20. Einrichtung nach Anspruch 19, **gekennzeichnet durch** einen mit dem Antrieb (95) wirkverbundenen Drehgeber (96), mittels welchem die infolge der einzelnen Drehphasen (P1 bis P4) relativ zu der Ansaugöffnung (21) in axialer Richtung orientierten Bewegungsabläufe des Innenrohres (30) steuerbar sind.

21. Einrichtung nach Anspruch 20, **dadurch gekennzeichnet, dass** die infolge der Drehphasen (P1 bis P4) in axialer Richtung bewirkten Bewegungsabläufe des Innenrohres (30) von dem Drehgeber (96) und dem Antrieb (95), beispielsweise von einem elektromotorischen Antrieb derart gestoppt werden, dass das Innenrohr (30) in Bezug auf die Ansaugöffnung (21) des Aussenrohres (20) zwangsläufig in der Offenstellung angeordnet ist.
